# EUROPEAN PATENT SPECIFICATION

(11) **EP 3 781 701 B1**
(45) Date of publication and mention of the grant of the patent: **01.06.2022**
(21) Application number: 19718170.4
(22) Date of filing: 17.04.2019
(51) Int. Cl.: C12Q 1/04, C12Q 1/18

(54) **DETECTION OF BACTERIA**
NACHWEIS VON BAKTERIEN
DÉTECTION DE BACTÉRIES

(30) Priority: 19.04.2018 GB 201806361
(43) Date of publication of application: 24.02.2021
(73) Proprietor: Ecole Polytechnique Fédérale de Lausanne (EPFL), 1015 Lausanne (CH)
(72) Inventor: JOVIC, Milica, 1950 Sion (CH); LESCH, Andreas, 40125 Bologna (IT); ZHU, Yingdi, 1950 Sion (CH); GIRAULT, Hubert, 1088 Ropraz (CH); PICK, Horst, 1134 Vufflens-Chateau (CH)
(74) Representative: Hanson, William Bennett
(86) International application number: PCT/EP2019/059905
(87) International publication number: WO 2019/201986

(56) References cited:
- FIDEL G PEREZ ET AL: "Immunomagnetic Separation with Mediated Flow Injection Analysis Amperometric Detection of Viable Escherichia coli O157", ANALYTICAL CHEMISTRY, vol. 70, no. 11, 24 April 1998 (1998-04-24), pages 2380-2386, XP055587689,
- LUKAS NEJDL ET AL: "Remote-controlled robotic platform ORPHEUS as a new tool for detection of bacteria in the environment : Microfluidics and Miniaturization", ELECTROPHORESIS, vol. 35, no. 16, 13 March 2014 (2014-03-13), pages 2333-2345, XP055603212, ISSN: 0173-0835, DOI: 10.1002/elps.201300576
- ISMAIL H BOYACI ET AL: "Amperometric determination of live Escherichia coli using antibody-coated paramagnetic beads", ANALYTICAL AND BIOANALYTICAL CHEMISTRY, SPRINGER, BERLIN, DE, vol. 382, no. 5, 29 June 2005 (2005-06-29) , pages 1234-1241, XP019327458, ISSN: 1618-2650, DOI: 10.1007/S00216-005-3263-8
- NGA-CHI YIP ET AL: "Real-time electrocatalytic sensing of cellular respiration", BIOSENSORS AND BIOELECTRONICS, vol. 57, 11 February 2014 (2014-02-11), pages 303-309, XP055156142, ISSN: 0956-5663, DOI: 10.1016/j.bios.2014.01.059
- JUSTIN D. BESANT ET AL: "Rapid electrochemical phenotypic profiling of antibiotic-resistant bacteria", LAB ON A CHIP, vol. 15, 13 May 2015 (2015-05-13), pages 2799-2807, XP055407704, ISSN: 1473-0197, DOI: 10.1039/C5LC00375J

## Description

### Background to the Invention

This invention relates to a method for the electrochemical detection of viable bacteria in a sample.

Bacterial detection and the determination of the antimicrobial susceptibility pattern represent a cornerstone of clinical microbiology. Bacterial detection methods can be grouped into genotypic techniques that profile an organism's genetic material (primarily its DNA) and phenotypic techniques that profile an organism's metabolic attributes. The genotypic methods include techniques such as real-time polymerase chain reaction (PCR), fluorescence *in situ* hybridization (FISH) and 16S ribosomal RNA gene sequencing, which are independent of the physiological state of the organism and which are not influenced by the composition of the growth medium or by the organism's growth phase.^{9, 21, 24} However, the stability and the reproducibility of these methods can barely reach the requirements for routine clinical diagnosis. Phenotypic techniques provide understanding of the physiological and functional activities of the organism at the protein level. The predominant proteomic phenotypic technologies include Matrix Assisted Laser Desorption Ionisation-Time of flight-Mass Spectrometry (MALDI-TOF-MS), ElectroSpray Ionisation-Mass Spectrometry (ESI-MS), Surface Enhanced Laser Desorption Ionisation (SELDI) and Sodium DodecylSulfate-Polyacrylamide Gel Electrophoresis (SDS-PAGE).⁹ Indeed, the main drawback of all these technologies is the culture-based approach, meaning that a lengthy culture enrichment is still necessary prior to the bacteria detection.

Antimicrobial susceptibility testing (AST) of bacterial pathogens for the determination of the antimicrobial susceptibility profile is considered as the gold standard in hospitals and microbiology laboratories, since the antimicrobial resistance (AMR) is a major threat to the public health worldwide. It is estimated that around 700,000 deaths occur annually due to the AMR.^{21, 22, 24-26} In hospitals, AST is conducted using phenotypic antimicrobial drug culture approaches like the broth (or agar) microdilution method, disk diffusion method, gradient tests, agar dilution and breakpoint tests, which are based on the continuous exposure of a bacterial isolate to a set of antimicrobials followed by the optical detection of the growth.²⁴ Standard AST requires 1 - 3 days in order to allow the bacteria to multiply to detectable levels (culture enrichment). After bacteria pre-culture (hours or days), additional 12 - 24 hours are required to perform the susceptibility tests.²⁵ Until the AST results are received, the patients are usually treated with broad-spectrum antibiotics to cover the most probable pathogens. This procedure can result in incorrect initial therapy leading potentially to further infection and increased mortality.

Other techniques have been developed for the detection of the antimicrobial resistance including nucleic acid staining²⁹, nanoliter optical sensing of stochastic confinement¹³, motion detector using atomic force microscopy¹⁷, mass monitoring of bacterial growth using cantilevers¹² and surface plasmon resonance⁵. Molecular diagnostic assays that target DNA and genetic mutations associated with drug resistance provide faster diagnostics but require pre-knowledge with respect to the genetic basis of the antibiotic resistance for a target pathogen. Polymerase chain reaction (PCR)-based assays have complex nucleic acid amplification steps, and false positive and negative results may be obtained due to the amplification of complex matrices and enzymatic inhibition from different blood and urine components.⁹ Almost all of these techniques require bulky and complex equipment.

Bacterial viability assays have been employed for decades to provide a simple, reliable and cost-effective detection of metabolically-active cells, either by direct¹⁴ or indirect measurements of bacterial metabolites. Indirect methods include the monitoring of oxygen consumption in the bacteria medium or monitoring the oxidation state of redox markers (e.g. ferricyanide^{6, 11, 19}, resazurin², tetrazolium salts^{2, 20, 23, 27}). For the latter approach, bacteria are incubated together with the redox marker in physiological solution. If bacterial cells are metabolically active their metabolites create a chemically reducing environment that will reduce the oxidation state of the redox marker. This change can be followed using spectroscopic methods (UV-Vis absorbance or fluorescence) or by electrochemical methods (voltammetry). Recently, viability assays have been adapted for antimicrobial susceptibility testing, where an antibiotic is added to the medium containing bacteria and the redox marker. In such cases, the resistant bacteria continue to multiply and metabolize the redox marker, i.e. reducing the oxidation state of the redox marker, while susceptible bacteria do not.^{2,} 10

The sensitivity of the bacterial viability assays depends on the requirement of a sufficiently detectable quantity of the reduced form of the redox marker, which is directly related to the bacteria concentration and the reaction time of bacteria with the redox marker. For example, bacterial viability assays performed in microtiter plate formats (consuming microliter volumes) can require up to 8 h for the detection of low bacteria concentration, i.e. few cells ·mL⁻¹.

Indeed, different strategies have been followed to improve the sensitivity of bacterial viability assays. One strategy is based on increasing the local concentration of bacteria by using microfluidic devices in which bacteria are confined in microliter or nanoliter volumes.^{2, 3, 8, 16, 30} Despite this, the required time for analysis remains long (i.e. several hours). Very sensitive optical techniques for microfluidic devices operate with samples divided into nanoliter droplets inside a microfluidic channel where the signal is gathered sequentially from each droplet with a high-powered fluorescence microscope.^{3, 7, 15} Plenty of the microfluidic devices detect either the presence of bacteria or the antimicrobial susceptibility profile.^{2, 15, 28, 31}

Another strategy to improve the sensitivity of bacteria viability assays is to employ bacteria enrichment/ separation and culture-independent immunological methods. These methods have been successfully used to improve the sensitivity for bacteria detection using MALDI-TOF-MS and fluorescence spectroscopy. For instance, Fe₃O₄ nanoparticle-graphene composite nanosheets decorated with chitosan were used to capture *Pseudomonas aeruginosa* and *Staphylococcus aureus* and perform the bacteria detection using fluorescence spectroscopy or MALDI-TOF-MS analysis.¹ Commercial anti*-Salmonella* Dynabeads^{®} (Lake Success, USA) were used to of detection (LOD) being 107 cells ·mL⁻¹ in water and 109 cells ·mL⁻¹ in human urine or chicken blood.¹⁸ Janus magnetic mesoporous silica nanoparticles coated with anti-*E. coli* antibodies were used for foodborne bacterial enrichment and fluorescent identification of E. *coli* that can be combined with MALDI-TOF-MS.⁴

Fidel G Perez et al: "Immunomagnetic Separation with Mediated Flow Injection Analysis Amperometric Detection of Viable Escherichia coli O157", Analytical Chemistry, vol. 70, no. 11, 24 April 1998, pages 2380-2386, describes use of a magnetic bead-based immunoassay coupled with amperometric flow detection analysis (FIA). Antibody-modified Dynabeads were used to capture *E. Coli* from the matrix solution. The electrochemical response of viable captured bacteria was evaluated in the presence of two different mediators.

Lukas Nejdl et al: "Remote-controlled robotic platform ORPHEUS as a new tool for detection of bacteria in the environment: Microfluidics and Miniaturization" Electrophoresis, vol. 35, no. 16, 1 August 2014, pages 2333-2345, describes a 3D printed flow chip applied for detection of S. *aureus* based on production of the ALP enzyme by S. *aureus.* Bacteria were captured on modified magnetic particles and the electrochemical detection step was based on the enzymatic cleaving of electrochemically inactive 1-naphtyl phosphate to electrochemically active 1-naphtol.

Ismail H Boyaci et al: "Amperometric determination of live Escherichia coli using antibody-coated paramagnetic beads", Analytical and Bioanalytical Chemistry, vol. 382, no. 5, 1 July 2005, pages 1234-1241, describes detection of *E. coli* using a magnetic bead-based immunoassay in which magnetic beads were used for capturing, incubation and separation.

Nga-Chi Yip et al: "Real-time electrocatalytic sensing of cellular respiration", Biosensors and Bioelectronics, vol. 56, 11 February 2014, pages 303-309, describes a real-time electrochemical mediator assay to enable the assessment of cell numbers and chemical toxicity, based on determination of oxygen using an electrochemical mediator.

Justin D. Besant et al: "Rapid electrochemical phenotypic profiling of antibiotic-resistant bacteria", Lab on a Chip, vol. 15, 1 January 2015, pages 2799-2807, describes monitoring the electrochemical reduction of a redox-active molecule to reports on levels of metabolically-active bacteria.

### Summary of the Invention

The present invention relates to a method based on an electrochemical bacterial viability assay with a highly efficient affinity bacteria enrichment/ separation in form of a bacteria isolate (super)paramagnetic agglutinate on or close to the detection electrode.

The invention provides a method according to claim 1. The method provides a significant reduction of bacteria culturing time and very sensitive detection of the oxidation state of the metabolic redox marker, which is directly linked to the number of viable bacteria in the sample. The method can be applied for total or species-specific bacteria detection and bacteria quantification (bacterial counts). If an antibiotic is added to the sample at any stage, such as before bacteria affinity enrichment/separation, the present method can be used to perform antimicrobial susceptibility testing (AST). Optional features of the invention are set out in the dependent claims.

### Brief Description of the Drawings

The invention will now be described in more detail, by way of example only, with reference to the accompanying drawings, in which:
**Fig. 1a** schematically shows a method for the electrochemical viability assay based on a highly efficient affinity enrichment/ separation of bacteria from a liquid sample;
**Fig. 1b** schematically shows details of steps in the method of **Fig. 1a** preceding the bacteria isolate (super)paramagnetic agglutinate formation;
**Fig. 2a** shows a typical differential pulse voltammogram illustrating the electrochemical detection of Gram-negative bacteria (E. *coli);*
**Fig. 2b** shows a typical differential pulse voltammogram illustrating the electrochemical detection of Gram-positive bacteria *(B. subtilis);*
**Fig. 2c** illustrates the detection of E. *coli* by using ferricinium methanol as the redox marker;
**Fig. 2d** shows a typical linear sweep voltammogram illustrating the detection of E. *coli* by using a microelectrode and ferricinium methanol as the redox marker;
**Fig. 3a** shows the bacterial count curve for E. *coli* (strain DH5a) obtained by using a fluorometric bacteria viability assay;
**Fig. 3b** shows the bacterial count curve for E. *coli* obtained using the method presented in **Fig. 1****;**

### Detailed Description of Particular Embodiments

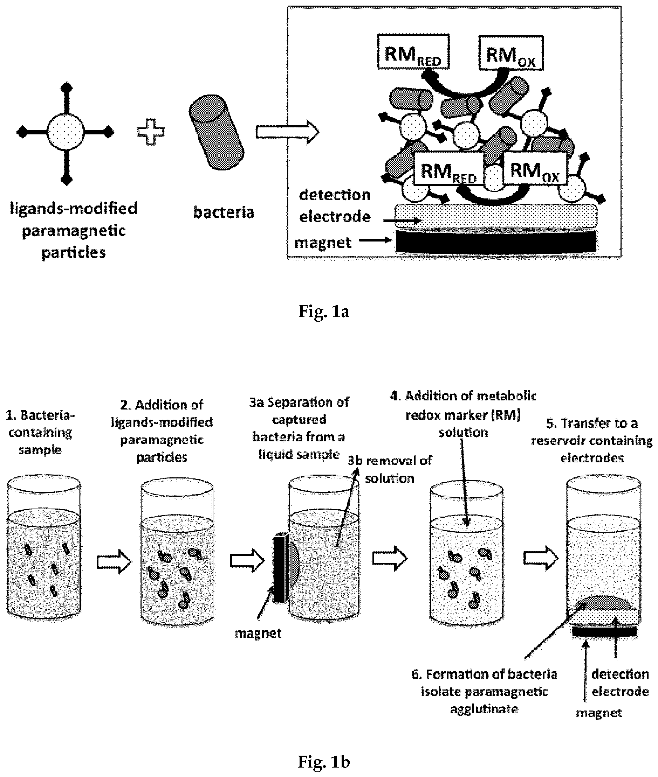

**Figs. 1a and 1b** schematically illustrate the principle of the present invention. The method incorporates a highly efficient affinity enrichment/ separation of bacteria from a liquid sample by using ligands-modified paramagnetic or superparamagnetic particles. Bacteria isolate-ligands-modified (super)paramagnetic particles are then incubated with a solution containing the metabolic redox marker in its oxidized form (RMox) prior to the transfer into the reservoir containing a detection electrode with a magnet positioned outside the reservoir, but close enough to the electrode, to accumulate the (super)paramagnetic particles and form a bacteria isolate (super)paramagnetic agglutinate on or close to the detection electrode (**Fig. 1b**). Bacteria metabolites present in the agglutinate react with the metabolic redox marker with higher oxidation state (i.e. in oxidized form) RMox and reduce them to a lower oxidation state (i.e. reduced form) RM_{RED}. The RMox molecules or ions that were not reduced by the bacteria are then electrochemically reduced at the detection electrode using a competitive mode: the more viable bacteria, the more RM_{RED} is produced and the less RMox is electrochemically detected. The present invention can be employed for both qualitative and quantitative bacteria analysis. The method can be used for the detection of total viable bacteria when non-specific ligands are used or for the detection of certain bacteria species when bacteria species-specific ligands are used. Furthermore, if an antibiotic is added to the sample at any stage, preferably before bacteria affinity enrichment/separation, the electrochemical read-out can be used for assessing the antimicrobial susceptibility profile: susceptible bacteria killed by the antibiotic do not reduce RM_{OX}, while resistant bacteria reduce RMox.

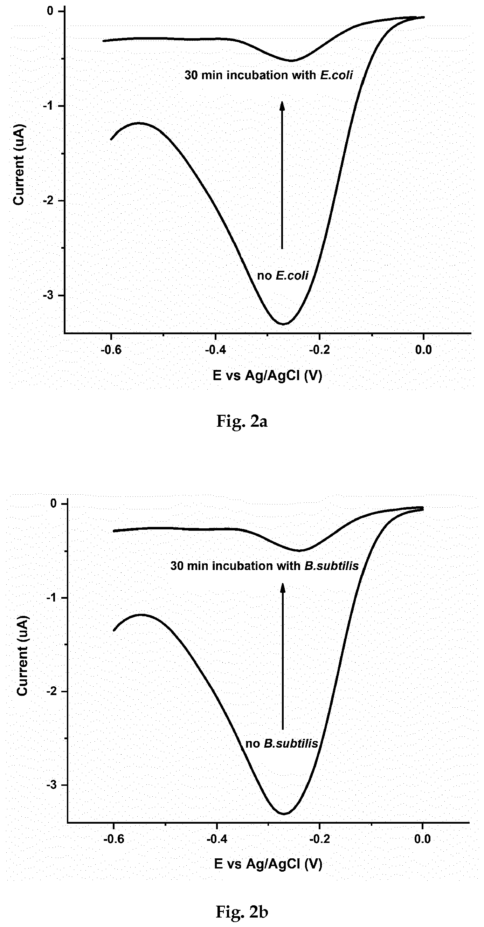

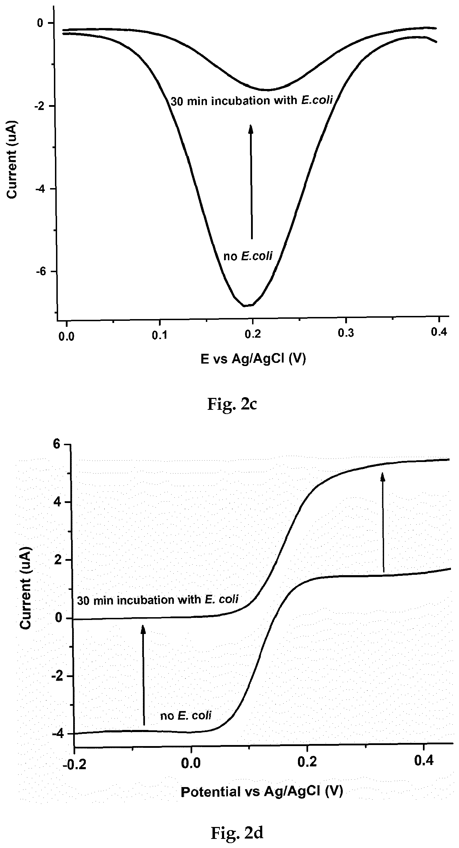

**Figs. 2a****-c** shows exemplary differential pulse voltammograms (DPV) illustrating the detection of Gram-negative (E. *coli,* 5×10⁸ cells ·mL⁻¹, redox markers resazurin and ferricinium methanol, **Fig. 2a** and **Fig. 2c****,** respectively) and Gram-positive bacteria *(B. subtilis,* 5×10⁸ cells ·mL⁻¹, redox marker resazurin, **Fig. 2b****)** by using the method presented in **Fig. 1****.** DPV parameters for resazurin were the following: potential range 0.0 V to -0.6 V vs. Ag/AgCl quasi-reference electrode, potential step 0.01 V, potential pulse 0.1 V, pulse time 0.05 s and the scan rate 0.02 V/s. DPV parameters for ferricinium methanol were the following: potential range 0.4 V to 0 V vs. Ag/ AgCl quasi-reference electrode, potential step 0.01 V, potential pulse 0.1 V, pulse time 0.05 s and the scan rate 0.02 V/s. Carbon nanotubes were used to form the working electrode, Ag/ AgCl served as a quasi-reference electrode and a second carbon layer served as a counter electrode. The supporting electrolyte was Luria-Bertani medium. **Fig. 2d** shows a linear sweep voltammogram (LSV) illustrating the detection of E. *coli* (5×10⁸ cells ·mL⁻¹), recorded with a platinum disk microelectrode, 25 µm in diameter, surrounded by an insulating glass sheath in a ferricinium methanol - ferrocene methanol mixture (ratio 4:1). The LSV parameters were the following: potential range 0.5 V to -0.2 V vs. Ag-QRE, potential step 0.005 V and potential scan rate 0.01 V/s, Ag wire as quasi-reference electrode and a platinum wire as a counter electrode.

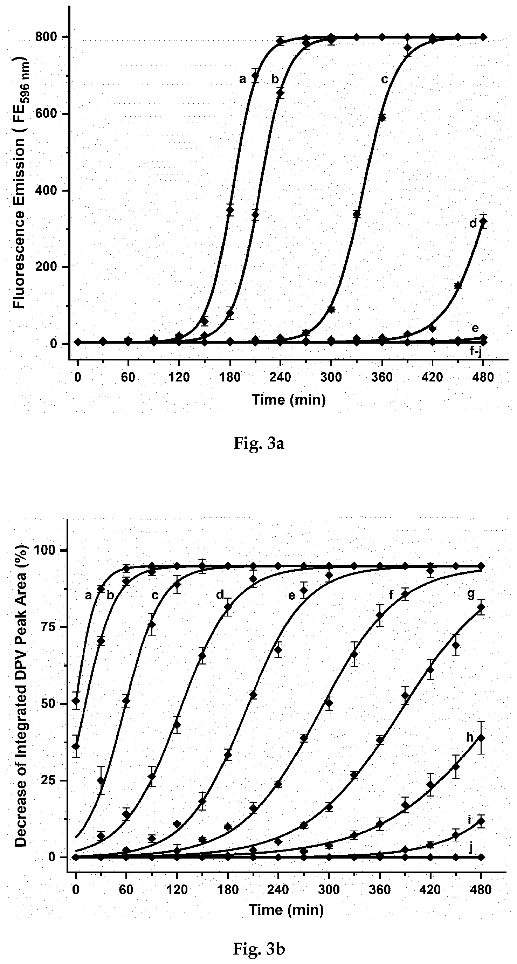

**Fig. 3** shows the results of quantitative bacteria analysis (bacterial counts) for *E. coli* obtained by using the fluorometric bacteria viability assay **(****Fig. 3a****)** and by using the present invention **(****Fig. 3b****).** The concentration range of E. *coli* was: a) 5×10⁸, b) 5×10⁷, c) 5×10⁶, d) 5×10⁵, e) 5×10⁴, f) 5×10³, g) 5×10², h) 50, i) 5 and j) 0 cells ·mL⁻¹. Both methods used the same redox marker (i.e. resazurin) and the readout was conducted every 30 min until 8 h.

**Table 1** shows the antimicrobial susceptibility testing (AST) report for multiresistant strain of E. *coli* DH5a (concentration 5×10⁵ cells ·mL⁻¹) obtained by using the standard automatic phenotypic system based on the broth dilution method (bioMérieux Vitek 2 system) and by using the method presented in **Fig. 1****.** Nine different antibiotics were tested in the following concentrations: 0 (control), 1, 2, 4, 8, 16, 32 and 64 µg ·mL⁻¹. The resistance phenotype was confirmed and interpreted according to the Clinical and Laboratory Standards Institute (CLSI) guidelines, version 2017. MIC - presents minimum inhibitory concentration, the lowest antibiotic concentration that can inhibit the growth of bacteria; R - resistant bacteria, S - susceptible bacteria.

**Table 2** shows the antimicrobial susceptibility testing (AST) report for the blood sample containing the multi-resistant E. *coli* strain DH5a, obtained by using the method presented in **Fig. 1****.** Nine different antibiotics were used in the following concentrations: 0 (control), 1, 2, 4, 8, 16, 32 and 64 µg ·mL⁻¹. The resistance phenotype was confirmed and interpreted according to the Clinical and Laboratory Standards Institute (CLSI) guidelines, version 2017. MIC - presents minimum inhibitory concentration, the lowest antibiotic concentration that can inhibit the growth of bacteria; R - resistant bacteria, S - susceptible bacteria.

### Results

**Fig. 2** shows that the present invention can be employed for qualitative bacteria analysis when using bacteria species-specific ligands. Paramagnetic particles coated with anti-E. *coli* antibodies (Abs) and anti-B. *subtilis* Abs were employed for the specific capturing of E. *coli* and *B. subtilis* and the representative differential pulse voltammograms (DPVs) obtained at macroelectrodes by using resazurin as the redox marker are presented in **Fig. 2a** and **Fig. 2b****,** respectively. The DPV obtained for the detection of E. *coli* at macroelectrodes by using ferricinium methanol as the redox marker is presented in **Fig. 2c****.** A linear sweep voltammogram (LSV) at microelectrodes for the detection of E. *coli* by using ferricinium methanol as the metabolic redox marker is presented in **Fig. 2d****.** The shapes of all of the voltammograms clearly indicate the differences between the blank samples and the samples containing specific bacteria. When the targeted bacteria species were present in the sample and captured by the species-specific ligand, it reduced a certain amount of RMox so that a lower amount of RMox was detected by the electrochemical reduction at the detection electrode, leading therefore to the decrease in the reduction/peak current (DPV) or reduction plateau current (LSV). Furthermore, due to the small finite volume of the redox marker solution in the electrochemical cell, coulometric measurements rather than amperometric measurements can be carried out.

**Fig. 3** shows that the present invention can be employed for quantitative bacteria analysis (bacterial counts). The presented method was compared with the standard commercial fluorometric bacteria viability assay (alamarBlue^{™} Cell Viability, ThermoFischer) for quantitative analysis of E. *coli* (strain DH5a). The obtained results indicate that the present method allows the detection of 5 millions of bacteria per mL within 1 hour **(****Fig. 3b****)** rather than 4 hours that are required for the standard fluorometric method **(****Fig. 3a****).**

**Table 1** shows that the present invention can be employed for the assessment of the antimicrobial susceptibility from pure bacteria cultures. The method was compared with a standard phenotypic system used in hospitals worldwide (bioMérieux Vitek 2 system) for antimicrobial susceptibility testing of the same multi-resistant E. *coli* strain DH5a. Due to the bacteria enrichment strategy and the sensitive electrochemical detection, the presented method provides the antimicrobial susceptibility testing report in 2.5 h instead of >9 h, as required for the Vitek 2 system.

**Table 1**

| **Antibiotic** | **bioMerieux Vitek 2** | | **Present method** | |
|---|---|---|---|---|
| | **MIC (µg ·mL⁻¹)** | **Interpretation** | **MIC (µg ·mL⁻¹)** | **Interpretation** |
| Ampicillin | >=32 | R | 32 | R |
| Cefuroxime | >=64 | R | 64 | R |
| Cefpodoxime | >=8 | R | 16 | R |
| Ceftriaxone | >=64 | R | 64 | R |
| Gentamicin | >=16 | R | 16 | R |
| Ciprofloxacin | >=4 | R | 8 | R |
| Tetracyclin | <=1 | S | 1 | s |
| Nitrofurantoin | <=16 | S | 8 | S |
| Fosfomycin | <=16 | S | 8 | S |

**Table 2** shows that the present invention can be employed for the assessment of the antimicrobial susceptibility directly from blood samples. Thanks to the highly efficient affinity enrichment/separation of bacteria from the blood samples, it was possible to start the antimicrobial susceptibility testing after only 6 h of blood culturing. Normally, the antimicrobial susceptibility testing is started when the blood bottles turn positive, which usually requires days of blood culturing. Furthermore, the antimicrobial susceptibility testing report was provided in 2.5 h, with an overall time from the blood sampling to the AST result being only 8.5 h.

**Table 2**

| **Antibiotic** | **Present method** | |
|---|---|---|
| | **MIC (µg ·mL⁻¹)** | **Interpretation** |
| Ampicillin | 32 | R |
| Cefuroxime | 64 | R |
| Cefpodoxime | 16 | R |
| Ceftriaxone | 64 | R |
| Gentamicin | 16 | R |
| Ciprofloxacin | 16 | R |
| Tetracyclin | 2 | S |
| Nitrofurantoin | 8 | S |
| Fosfomycin | 16 | S |

### Advantages of the present invention

Bacterial viability assays have been employed for decades for bacteria detection based on the direct or indirect measurement of bacteria metabolites. For the indirect method, bacteria are incubated in solution together with a metabolic redox marker and if bacterial cells are metabolically active their metabolites create a reducing environment that will cause a change in the oxidation state of the redox marker that can be followed by spectroscopic or electrochemical methods. The principle is simple but challenging, because of limitations on achieving the necessary sensitivity due to the requirement of lengthy culture-based enrichment procedures. By using the present invention, bacterial cells are first captured from the sample by ligands-modified paramagnetic or superparamagnetic particles and by using an external magnet are then transferred into a liquid medium containing the redox marker in low concentrations. Due to the highly efficient bacteria enrichment/ separation step, long bacteria culturing times are reduced or even no longer necessary, depending on the bacteria concentration in the sample. Furthermore, the captured bacteria (super)paramagnetic particles are then introduced into the reservoir containing the electrodes and the magnets positioned so as to localize the bacterial metabolites on or close to the detection electrode's surface and to provide a very sensitive detection of the redox marker as a direct measure of bacterial viability. Accordingly, the present invention can be used for total or species-specific bacteria detection and bacteria quantification (bacterial counts). Moreover, if an antibiotic is added in the sample medium at any stage, such as before bacteria affinity enrichment/ separation, the invention can be used for the detection of antimicrobial resistance. Furthermore, the electrochemical detection can operate in dark and turbid samples where spectroscopic detection methods are hindered.

### Examples

### Example 1 - Bacteria detection using the electrochemical viability assay with highly efficient affinity enrichment/separation

*Escherichia coli (E. coli)* strain DH5a and *Bacillus subtilis (B. subtilis)* strain 168 were grown as pre-cultures in Luria-Bertani medium at 37 °C for 6 h with continuous shaking at 200 rpm. 100 µL of each pre-culture was added into 3 mL of Luria-Bertani medium and incubated overnight at 37 °C with continuous shaking at 200 rpm. The obtained fresh cultures were afterwards used as stock solution for further experiments. Concentrations of bacterial cells in Luria-Bertani medium were determined by measuring the optical density at 600 nm (OD₆₀₀ₙₘ). 1.0 OD₆₀₀ₙₘ corresponds to ~ 8×10⁸ cells ·mL⁻¹.

In order to demonstrate the feasibility of the present method for bacteria detection, two model solutions were prepared: the first solution containing 5×10⁸ 5×10⁸ cells ·mL⁻¹ of E. *coli* in Luria-Bertani medium and the second solution containing 5×10⁸ cells ·mL⁻¹ of *B. subtilis* in Luria-Bertani medium. Protein A/G-coated superparamagnetic beads (MBs, Thermo Scientific) were modified with anti-E. *coli* (2 mg ·mL⁻¹, polyclonal, IgG isotype, Abcam) and anti-B. *subtilis* antibodies (4 mg ·mL⁻¹, polyclonal, IgG isotype, Abcam) and washed with phosphate buffered saline containing 0.05% (V/V) of Tween 20. The mixture was incubated for 1 h at room temperature with continuous shaking and transferred into 1 mL of the model solutions containing E. *coli* or *B. subtilis.* After incubation with continuous shaking for 30 min at 37°C, the superparamagnetic beads with captured bacterial cells were collected using a magnetic separation stand.

The collected superparamagnetic complexes were then incubated with 25 µL of Luria-Bertani medium containing redox marker (1 mM resazurin, 2 mM ferricinium methanol or a 2 mM mixture of ferricinium methanol and ferrocene methanol (e.g. in a ratio of 4:1)) at 37°C for 25 min. After incubation, the mixture was transferred into a reservoir of a microchip. The microchip was composed of eight reservoirs, each containing an inkjet-printed carbon nanotube working electrode (WE), Ag/AgCl quasi-reference electrode (QRE) and a second carbon layer as a counter electrode (CE). The microchip was placed in a holder containing eight integrated magnets that are located below the working electrode of each reservoir, separated by the microchip plastic support. In this way, the bacteria cells captured by the superparamagnetic beads were concentrated closely to the working electrode surface. The electrochemical read-out was conducted by employing differential pulse voltammetry (DPV) at carbon nanotube (CNT) macroelectrodes and linear sweep voltammetry (LSV) at platinum microelectrodes. DPV parameters for resazurin were as follows: potential range 0.0 V to -0.6 V vs. Ag/ AgCl quasi-reference electrode, potential step 0.01 V, potential pulse 0.1 V, pulse time 0.05 s and the scan rate 0.02 V/s. DPV parameters for ferricinium methanol were as follows: potential range 0.4 V to 0 V vs. Ag/AgCl quasi-reference electrode, potential step 0.01 V, potential pulse 0.1 V, pulse time 0.05 s and the scan rate 0.02 V/s. LSV parameters for ferricinium methanol were as follows: potential range 0.5 V to -0.2 V vs. a Ag quasi-reference electrode, potential step 0.005 V and potential scan rate 0.01 V/s. The recorded differential pulse voltammograms for the samples with E. *coli* and *B. subtilis* obtained by using resazurin as the redox marker are presented in **Fig. 2a** and **Fig. 2b****,** respectively; the differential pulse voltammogram for the sample with E. *coli* obtained by using ferricinium methanol as the redox marker is presented in **Fig. 2c****;** the linear sweep voltammogram for a sample with E. *coli* obtained by using an 4:1 ferricinium methanol:ferrocence methanol mixture as the redox marker system is presented in **Fig. 2d****.** The plots clearly indicate the differences between the blank samples and the samples containing specific bacteria species.

### Example 2 - Bacterial counts - quantitative analysis of microbes using the electrochemical viability assay with highly efficient affinity enrichment/separation

Bacterial counts were conducted using the standard fluorometric method and the present method. The fluorometric method was adopted from the protocol of alamarBlue^{™} Cell Viability kit by ThermoFischer. E. *coli* strain DH5a was cultured as described in *Example 1.* The bacteria were incubated with 1 mM of resazurin at 37°C. The cell concentrations were set as 5×10⁸, 5×10⁷, 5×10⁶, 5×10⁵, 5×10⁴, 5×10³, 5x102, 50, 5 and 0 cells ·mL⁻¹, obtained by 10-fold serial dilution of the E. *coli* stock solution. The fluorescence emission at 590 nm of each solution was measured after every 30 min of incubation, which was carried out continuously for 8 h. The fluorescence measurement was conducted with a PerkinElmer LS 55 fluorescence spectrometer. The instrumental parameters were set as excitation wavelength at 540 nm, emission wavelength window from 555 nm to 700 nm, excitation slit 4.4 nm, emission slit 4.4 nm and low gain. Bacterial counts obtained by the fluorometric method are presented in **Fig. 3a****.**

Bacteria counts were conducted also by using the present method based on bacteria species-specific enrichment/separation. As described in *Example* 1, protein A/G-superparamagnetic beads were coated with anti-E. *coli* antibodies and transferred into 1 mL of Luria-Bertani medium containing different concentrations of *E. coli* (5×10⁸, 5×10⁷, 5×10⁶, 5×10⁵, 5×10⁴, 5×10³, 5×10², 50, 5 and 0 cells ·mL⁻¹), obtained by 10-fold serial dilution of the stock solution. After incubation with continuous shaking for 30 min at 37°C, the superparamagnetic beads with captured E. *coli* were collected using the magnetic stand.

The collected complexes for each bacteria concentration were incubated with 25 µL of Luria-Bertani medium containing the redox marker (1 mM resazurin) at 37°C for 25 min. After incubation, the mixture was transferred into a reservoir of a microchip where the bacteria cells captured by superparamagnetic beads were concentrated closely to the working electrode surface (as described in *Example 1*). The electrochemical read-out was conducted every 30 min for up to 8 h by employing differential pulse voltammetry (DPV). Obtained bacterial counts show that the present method allows the detection of 5 millions of bacteria per mL within 1 h rather than 4 h that are required for the fluorometric method, as shown in **Fig. 3b****.**

### Example 3 - Antimicrobial susceptibility testing (AST) of pure bacteria cultures by using the electrochemical viability assay with highly efficient affinity enrichment/separation

The multi-resistant E. *coli* strain DH5a was provided by the Hospital of Valais, Sion, Switzerland. For each bacteria sample, bacterial cells were harvested from the growth medium by centrifugation at 7,500 g for 3 min, then washed two times with deionized water and suspended in deionized water (18.2 MΩ cm) to reach a concentration of ~ 5×10⁵ cells ·mL⁻¹ in Luria-Bertani medium. A detailed antimicrobial susceptibility profile of the E. *coli* strain was obtained by using the bioMerieux VITEK 2 automated AST system in Hôpital du Valais, Sion, Switzerland **(Table 1).**

Antimicrobial susceptibility testing of the same E. *coli* strain was conducted using the present method. Briefly, in each Eppendorf tube containing 1 mL of 5×10⁵ cells ·mL⁻¹ a certain concentration of an antibiotic was added at 37°C and incubated with continuous shaking for 1 h. Nine different antibiotics were used for the test: ampicillin, cefuroxime, cefpodoxime, ceftriaxone, gentamicin, ciprofloxacin, tetracycline, nitrofurantoin and fosfomycin (Sigma-Aldrich). Based on the broth dilution method, the antibiotic concentrations in different tubes were: 0 (control), 1, 2, 4, 8, 16, 32 and 64 µg ·mL⁻¹. After the first incubation, superparamagnetic beads (10 mg ·mL⁻¹) conjugated with the anti-E. *coli* antibodies were added into each tube to capture viable E. *coli* cells and incubated under continuous shaking at 37°C for 30 min. Afterwards, the superparamagnetic beads with captured viable E. *coli* cells were collected and the supernatant was discarded. The collected complex was incubated with 25 µL of the redox marker solution (200 µM resazurin in Luria-Bertani medium) at 37°C for 25 min and then introduced into the reservoir of the microchip (as described in *Example 1*). In order to obtain the antimicrobial susceptibility profile, the electrochemical read-out was conducted by employing differential pulse voltammetry (DPV). The DPV signal obtained from a measurement with the antibiotics was compared with that without the antibiotic (the control). If the former is the same as the latter, bacteria were defined as resistant against that concentration of the antibiotic. Otherwise, the bacteria were defined as susceptible to that concentration of the antibiotic. The resistance phenotype was confirmed and interpreted according to the Clinical and Laboratory Standards Institute (CLSI) guidelines, version 2017. A detailed antimicrobial susceptibility testing report is shown in **Table 1.** The present method provides the AST results in 2.5 h instead of > 9 h required for the standard phenotypic system Vitek 2.

### Example 4 - Antimicrobial susceptibility testing (AST) for blood stream infections by using the electrochemical viability assay with highly efficient affinity enrichment/separation

The bacteria concentration in adult patients' blood with bloodstream infections is typically 1 - 100 CFU (colony forming unit) per mL. To simulate real diagnosis, 6 mL of whole blood collected from a patient was spiked with the multi-drug resistant *E. coli* cells (100 cells mL⁻¹), injected into a BacT/Alert^{®} FA Plus aerobic blood culturing bottle (containing 30 mL of broth) and cultured at 37°C with continuous shaking. After 6 h of incubation, 1 mL of the culture liquid was firstly centrifuged at 1200 rpm for 5 min to remove red blood cells; then at 13,000 rpm for 3 min to precipitate bacteria cells. The precipitated cells were re-suspended in 1 mL of Luria-Bertani medium and captured with the superparamagnetic beads conjugated with anti-E. *coli* antibodies. The captured bacteria complex was incubated at 37°C for 60 min with 24 µL of Luria-Bertani medium containing one antibiotic for each measurement. Nine different antibiotics were tested: ampicillin, cefuroxime, cefpodoxime, ceftriaxone, gentamicin, ciprofloxacin, tetracycline, nitrofurantoin and fosfomycin (Sigma-Aldrich). Based on the broth dilution method, the antibiotic concentrations were: 0 (control), 1, 2, 4, 8, 16, 32 and 64 µg ·mL⁻¹. After the incubation, 1 µL of the redox marker (5 mM resazurin) was added and the mixture was incubated for 30 min to allow the reduction of redox marker by living bacteria. The mixture was introduced into the reservoir of the inkjet-printed microchip (as described in the *Example 1).* The antimicrobial susceptibility profile was obtained as explained in the *Example* 3. With the present method it was possible to start AST after only 6 h of blood culturing. Afterwards, the AST results are obtained in 2.5 h with the overall time from the blood sample to the AST result being only 8.5 h **(Table 2).**

References:
1 H. N. Abdelhamid, and H-F. Wu, 'Multifunctional Graphene Magnetic Nanosheet Decorated with Chitosan for Highly Sensitive Detection of Pathogenic Bacteria', Journal of Materials Chemistry B, 1 (2013), 3950 - 61.
2 J. D. Besant, E. H. Sargent, and S. H. Kelley, 'Rapid Electrochemical Phenotypic Profiling of Antibiotic-Resistant Bacteria', Lab Chip, 15 (2015), 2799 - 807.
3 J. Q. Boedicker, L. Li, T. R. Kline, and R. F. Ismagilov, 'Detecting Bacteria and Determining Their Susceptibility to Antibiotics by Stochastic Confinement in Nanoliter Droplets Using Plug-Based Microfluidics', Lab Chip, 8 (2008), 1265 - 72.
4 Z-M. Chang, Z. Wang, D. Shao, J. Yue, M-m. Lu, L. Li, M. Ge, D. Yang, M-q. Li, H. Yan, Q. Xu, and W-f. Dong, 'Fluorescent-Magnetic Janus Nanorods for Selective Capture and Rapid Identification of Foodborne Bacteria', Sensors and Actuators B: Chemical, 260 (2018), 1004 -11.
5 Y.-L. Chiang, C.-H. Lin, M.-Y. Yen, Y.-D. Sue, S.-J. Chene, and H.-F. Chen,'Innovative Antimicrobial Susceptibility Testing Method Using Surface Plasmon Resonance.', Biosens. Bioelectron., 24 (2009), 1905 - 10.
6 K. Chotinantakul, W. Suginta, and A. Schulte, 'Advanced Amperometric Respiration Assay for Antimicrobial Susceptibility Testing', Analytical Chemistry, 86 (2014), 10315 - 22.
7 K. Churski, T. S. Kaminski, S. Jakiela, W. Kamysz, W. Baranska-Rybak, D. B. Weibel, and P. Garstecki, 'Rapid Screening of Antibiotic Toxicity in an Automated Microdroplet System.', Lab Chip, 12 (2012), 1629 - 37.
8 N. J. Cira, J. Y. Ho, M. E. Dueck, and D. B. Weibel, 'A Self-Loading Microfluidic Device for Determining the Minimum Inhibitory Concentration of Antibiotics', Lab Chip, 12 (2012), 1052 - 59.
9 D. Emerson, L. Agulto, H. Liu, and L. Liu, 'Identifying and Characterizing Bacteria in an Era of Genomics and Proteomics', BioScience, 58 (2008), 925.
10 P. Ertl, E. Robello, F. Battaglini, and S. R. Mikkelsen, 'Rapid Antibiotic Susceptibility Testing Via Electrochemical Measurement of Ferricyanide Reduction by Escherichia Coli and Clostridium Sporogenes', Analytical Chemistry, 72 (2000), 4957 - 64.
11 P. Ertl, B. Unterladstaetter, K. Bayer, and S. R. Mikkelsen, 'Ferricyanide Reduction by Escherichia Coli: Kinetics, Mechanism, and Application Ot the Optimization of Recombinant Fermentations', Analytical Chemistry, 72 (2000), 4949 - 56.
12 K. Y. Gfeller, N. Nugaeva, and M. Hegner, 'Rapid Biosensor for Detection of Antibiotic-Selective Growth of Escherichia Coli.', Appl. Environ. Microbiol., 71 (2005), 2626 - 31.
13 T. Gosiewski, M. Brzychczy-Wloch, A. Pietrzyk, A. Sroka, and M. Bulanda, 'Evaluation of the Activity of Thermostable DNA Polymerases in the Presence of Heme, as a Key Inhibitor in the Real Time Pcr Method in Diagnostics of Sepsis', Acta Biochim Pol, 60 (2013), 603 - 06.
14 R. Y. A. Hassan, M. M. Mekawy, P. Ramnani, and A. Mulchandani, 'Monitoring of Microbial Cell Viability Using Nanostructured Electrodes Modified with Graphene/ Alumina Nanocomposite', Biosensors and Bioelectronics, 91 (2017), 857 - 62.
15 D. K. Kang, M. M. Ali, K. Zhang, S. S. Huang, E. Peterson, M. A. Digman, E. Gratton, and W. Zhao, 'Rapid Detection of Single Bacteria in Unprocessed Blood Using Integrated Comprehensive Droplet Digital Detection.', Nat. Commun., 13 (2014), 5427.
16 S. H. Kelley, E. H. Sargent, and J. D. Besant, 'World Intellectual Property Organization Pat. Wo 2016/065475 A1'.
17 G. Longo, L. Alonso-Sarduy, L. Marques Rio, A. Bizzini, A. Trampuz, J. Notz, G. Dietler, and S. Kasas, 'Rapid Detection of Bacterial Resistance to Antibiotics Using Afm Cantilevers as Nanomechanical Sensors', Nat. Nanotechnol., 8,522 - 26.
18 A. J. Madonna, F. Basile, E. Furlong, and K. J. Voorhees, 'Detection of Bacteria from Biological Mixtures Using Immunomagnetic Separation Combined with Matrix-Assisted Laser Desorption/Ionization Time-of-Flight Mass Spectrometry.', Rapid Communications in Mass Spectrometry, 15 (2001), 1068 - 74.
19 T. S. Mann, and S. R. Mikkelsen, 'Antibiotic Susceptibility Testing at a Screen-Printed Carbon Electrode Array', Analytical Chemistry, 80 (2008), 843 - 48.
20 A. Martin, F. Portaels, and J. C. Palomino, 'Colorimetric Redox-Indicator Methods for the Rapid Detection of Multidrug Resistance in Mycobacterium Tuberculosis: A Systematic Review and Meta-Analysis', Journal of Antimicrobial Chemotherapy, 59 (2007), 175 - 83.
21 F. P. Maurer, M. Christner, M. Hentschke, and H. Rohde, 'Advances in Rapid Identification and Susceptibility Testing of Bacteria in the Clinical Microbiology Laboratory: Implications for Patient Care and Antimicrobial Stewardship Programs', Infectious Disease Reports, 9 (2017).
22 Jim O'Neill, 'Tackling Drug-Resistant Infections Globally: Final Report and Recommendations', The review on antimicrobial resistance (2016).
23 J-C. Palomino, A. Martin, M. Camacho, H. Guerra, J. Swings, and F. Portaels, 'Resazurin Microtiter Assay Plate: Simple and Inexpensive Method for Detection of Drug Resistance in Mycobacterium Tuberculosis', Antimicrobial Agents and Chemotherapy (2002), 2720 - 22.
24 R. P. Peters, M. A. van Agtmael, S. A. Danner, P. H. Savelkoul, and C. M. Vandenbroucke-Grauls, 'New Developments in the Diagnosis of Bloodstream Infections', Lancet Infect. Dis., 4 (2004), 751 - 60.
25 M. A. Pfaller, and R. N. Jones, 'Performance Accuracy of Antibacterial and Antifungal Susceptibility Test Methods: Report from the College of American Pathologists Microbiology Surveys Program (2001-2003).', Arch Pathol Lab Med., 130 (2006), 767 - 78.
26 A. Plüddemann, I. Onakpoya, S. Harrison, B. Shinkins, A. Tompson, R. Davis, C. P. Price, and C. Heneghan, 'Position Paper on Anti-Microbial Resistance Diagnostics', Centre for Evidence-Based Medicine Nuffield Department of Primary Care Health Sciences University of Oxford (2014).
27 S. N. Rampersad, 'Multiple Applications of Alamar Blue as an Indicator of Metabolic Function and Cellular Health in Cell Viability Bioassays', Sensors, 12 (2012), 12347 - 60.
28 M. Safavieh, M. U. Ahmed, M. Tolba, and M. Zourob, 'Microfluidic Electrochemical Assay for Rapid Detection and Quantification of Escherichia Coli.', Biosensors and Bioelectronics, 31 (2012), 523 - 28.
29 M. Safavieh, M. K. Kanakasabapathy, F. Tarlan, M. U. Ahmed, M. Zourob, W. Asghar, and H. Shaflee, 'Emerging Loop-Mediated Isothermal Amplification-Based Microchip and Microdevice Technologies for Nucleic Acid Detection', ACS Biomater. Sci. Eng., 14 (2016), 278 - 94.
30 P. Selvaganapathy, 'United States of America Pat. Us 2015/0118707 A1'.
31 M. Varshney, Y. Li, B. Srinivasan, and S. Tung, 'A Label-Free, Microfluidics and Interdigitated Array Microelectrode-Based Impedance Biosensor in Combination with Nanoparticles Immunoseparation for Detection of Escherichia Coli O157:H7 in Food Samples', Sensors and Actuators B, 128 (2007), 99 - 107.

## Claims

1. A method for the electrochemical detection of viable bacteria in a sample, the method comprising:
- affinity enrichment/ separation of bacteria from a liquid sample using ligand-modified paramagnetic or superparamagnetic particles, to form bacteria isolate-(super)paramagnetic complexes;
- placing the complexes together with a metabolic redox marker in solution into a container for incubation;
- subsequently transferring the solution containing the metabolic redox marker and complexes to an electrochemical cell having a detection electrode with an external magnet close to the detection electrode, such that the complexes form a bacteria isolate (super)paramagnetic agglutinate on or close to the detection electrode; and
- using an electrochemical read-out procedure to detect a change in oxidation state of the metabolic redox marker as a direct response to the metabolic activity of viable bacteria.

2. A method according to claim 1, wherein, in order to identify viable bacteria in the sample, the ligand-modified paramagnetic or superparamagnetic particles include bacteria species-specific ligands.

3. A method according to claim 2, wherein the bacteria species-specific ligands contain affinity labels, antibodies, single-stranded DNA aptamers or bacteriophages.

4. A method according to claim 1, wherein, in order to quantify the presence of viable bacteria in the sample in terms of end-point or kinetic assays, the ligand-modified paramagnetic or superparamagnetic particles include bacteria species non-specific ligands showing affinity to bacterial cells.

5. A method according to claim 4, wherein the non-specific ligands comprise lectins, mammalian immunoglobulins, antimicrobial peptides, carbohydrate-based polymers or biomimetic macromolecular constructs.

6. A method according to any preceding claim, wherein the electrochemical read-out procedure comprises an amperometric method, the detection electrode being used to measure a current, in association with a reference and/or counter electrode.

7. A method according to claim 6, wherein the electrochemical read-out procedure comprises steady-state voltammetry, linear sweep voltammetry or differential pulse voltammetry.

8. A method according to claim 6 or 7, wherein the detection electrode is one of: a screen-printed graphite or carbon electrode, an inkjet-printed carbon nanotube electrode, carbon, gold or platinum microelectrode, for example made by photolithography.

9. A method according to any preceding claim, wherein the sample is one of: blood, serum, plasma, urine, sputum, saliva, or interstitial fluid; in pure, diluted or any other processed liquid form.

10. A method according to any one of claims 1 to 8, wherein the sample is selected from a laboratory prepared bacteria culture, an environmental sample, an industrial sample, a food sample or a beverage sample.

11. A method according to any preceding claim, wherein the metabolic redox marker is a water-soluble substrate for a mitochondrial or cytoplasm enzyme produced by viable bacteria.

12. A method according to claim 11, wherein the metabolic redox marker is one of: a water soluble oxidised metallocene such as ferricinium methanol, an organometallic complex such as osmium tris-bipyridine, a redox dye such as a tetrazolium salt, resazurin, methylene blue or formazan.

13. A method according to any preceding claim, wherein one or more antibiotics are introduced into the sample, which contains bacteria, and wherein the change in oxidation state of the metabolic redox marker indicates if bacteria are resistant or susceptible to the one or more antibiotics.

14. A method according to claim 13, wherein the one or more antibiotics are in solid form, liquid form or solubilized and introduced to the sample before the affinity enrichment/ separation.

15. The method of claim 14, wherein the one or more antibiotics are coated on paper, nitrocellulose, a hydrogel, or a polymer.

## Patentansprüche

1. Verfahren zum elektrochemischen Nachweis von lebensfähigen Bakterien in einer Probe, wobei das Verfahren umfasst:
- Affinitätsanreicherung/Absonderung von Bakterien aus einer flüssigen Probe unter Verwendung von ligandenmodifizierten paramagnetischen oder superparamagnetischen Partikeln, um Bakterienisolat-(super)paramagnetische Komplexe zu bilden;
- Einbringen der Komplexe zusammen mit einem metabolischen Redoxmarker in Lösung in einen Behälter zur Inkubation;
- anschließendes Überführen der Lösung, die den metabolischen Redoxmarker und die Komplexe enthält, in eine elektrochemische Zelle, die eine Nachweiselektrode mit einem externen Magneten in der Nähe der Nachweiselektrode aufweist, so dass die Komplexe ein bakterienisoliertes (super)paramagnetisches Agglutinat auf oder in der Nähe der Nachweiselektrode bilden; und
- Verwendung eines elektrochemischen Ausleseverfahrens, um eine Änderung des Oxidationszustands des metabolischen Redoxmarkers als direkte Reaktion auf die metabolische Aktivität lebensfähiger Bakterien zu erfassen.

2. Verfahren nach Anspruch 1, wobei zur Identifizierung lebensfähiger Bakterien in der Probe die ligandenmodifizierten paramagnetischen oder superparamagnetischen Partikel bakterienspeziesspezifische Liganden enthalten.

3. Verfahren nach Anspruch 2, wobei die bakterienartspezifischen Liganden Affinitätsmarker, Antikörper, einzelsträngige DNA-Aptamere oder Bakteriophagen enthalten.

4. Verfahren nach Anspruch 1, **dadurch gekennzeichnet, dass** zur Quantifizierung der Anwesenheit lebensfähiger Bakterien in der Probe in Form von Endpunkt- oder kinetischen Assays die ligandenmodifizierten paramagnetischen oder superparamagnetischen Partikel bakterienspeziesunspezifische Liganden enthalten, die eine Affinität zu Bakterienzellen aufweisen.

5. Verfahren nach Anspruch 4, wobei die unspezifischen Liganden Lektine, Säugetier-Immunglobuline, antimikrobielle Peptide, Polymere auf Kohlenhydratbasis oder biomimetische makromolekulare Konstrukte umfassen.

6. Verfahren nach einem der vorhergehenden Ansprüche, wobei das elektrochemische Ausleseverfahren ein amperometrisches Verfahren umfasst, wobei die Nachweiselektrode zur Messung eines Stroms in Verbindung mit einer Referenz- und/oder Gegenelektrode verwendet wird.

7. Verfahren nach Anspruch 6, wobei das elektrochemische Ausleseverfahren Steady-State-Voltammetrie, Linear-Sweep-Voltammetrie oder Differential-Puls-Voltammetrie umfasst.

8. Verfahren nach Anspruch 6 oder 7, wobei die Nachweiselektrode eine der folgenden ist: eine siebgedruckte Graphit- oder Kohlenstoffelektrode, eine tintenstrahlgedruckte Kohlenstoffnanoröhrchen-Elektrode, eine Kohlenstoff-, Gold- oder Platin-Mikroelektrode, die beispielsweise durch Photolithographie hergestellt wurde.

9. Verfahren nach einem der vorhergehenden Ansprüche, wobei es sich bei der Probe um eine der folgenden Substanzen handelt: Blut, Serum, Plasma, Urin, Sputum, Speichel oder interstitielle Flüssigkeit; in reiner, verdünnter oder einer anderen verarbeiteten flüssigen Form.

10. Verfahren nach einem der Ansprüche 1 bis 8, wobei die Probe aus einer im Labor hergestellten Bakterienkultur, einer Umweltprobe, einer industriellen Probe, einer Lebensmittelprobe oder einer Getränkeprobe ausgewählt ist.

11. Verfahren nach einem der vorhergehenden Ansprüche, wobei der metabolische Redoxmarker ein wasserlösliches Substrat für ein mitochondriales oder cytoplasmatisches Enzym ist, das von lebensfähigen Bakterien produziert wird.

12. Verfahren nach Anspruch 11, wobei der metabolische Redoxmarker einer der folgenden ist: ein wasserlösliches oxidiertes Metallocen wie Ferrocenium-Methanol, ein metallorganischer Komplex wie Osmium-Tris-Bipyridin, ein Redoxfarbstoff wie ein Tetrazoliumsalz, Resazurin, Methylenblau oder Formazan.

13. Verfahren nach einem der vorhergehenden Ansprüche, wobei ein oder mehrere Antibiotika in die Probe, die Bakterien enthält, eingebracht werden und wobei die Änderung des Oxidationszustandes des metabolischen Redoxmarkers anzeigt, ob die Bakterien gegen das eine oder die mehreren Antibiotika resistent oder anfällig sind.

14. Verfahren nach Anspruch 13, wobei das eine oder die mehreren Antibiotika in fester Form, in flüssiger Form oder in gelöster Form vorliegen und der Probe vor der Affinitätsanreicherung/Absonderung zugeführt werden.

15. Verfahren nach Anspruch 14, wobei das eine oder die mehreren Antibiotika auf Papier, Nitrocellulose, ein Hydrogel oder ein Polymer aufgetragen sind.

## Revendications

1. Procédé pour la détection électrochimique de bactéries viables dans un échantillon, le procédé comprenant :
- l'enrichissement/la séparation sur la base de l'affinité de bactéries vis-à-vis d'un échantillon liquide en utilisant des particules paramagnétiques ou super-paramagnétiques modifiées en termes de ligand(s), afin de former des complexes super(paramagnétiques) d'isolat de bactéries ;
- le placement des complexes en association avec un marqueur redox métabolique en solution à l'intérieur d'un moyen de contenance pour une incubation ;
- subséquemment, le transfert de la solution qui contient le marqueur redox métabolique et les complexes jusqu'à une cellule électrochimique qui comporte une électrode de détection, un aimant externe étant situé à proximité de l'électrode de détection, de telle sorte que les complexes forment un agglutinat super(paramagnétique) d'isolat de bactéries sur l'électrode de détection ou à proximité de celle-ci ; et
- l'utilisation d'une procédure de lecture électrochimique pour détecter une modification de l'état d'oxydation du marqueur redox métabolique en tant que réponse directe à l'activité métabolique de bactéries viables.

2. Procédé selon la revendication 1, dans lequel, afin d'identifier les bactéries viables dans l'échantillon, les particules paramagnétiques ou super-paramagnétiques modifiées en termes de ligand(s) incluent des ligands spécifiques par rapport aux espèces de bactéries.

3. Procédé selon la revendication 2, dans lequel les ligands spécifiques par rapport aux espèces de bactéries contiennent des étiquettes d'affinité, des anticorps, des aptamères d'ADN à brin simple ou des bactériophages.

4. Procédé selon la revendication 1, dans lequel, afin de quantifier la présence de bactéries viables dans l'échantillon en termes d'essais de point final ou cinétiques, les particules paramagnétiques ou super-paramagnétiques modifiées en termes de ligand(s) incluent des ligands non spécifiques par rapport espèces de bactéries qui présentent une affinité vis-à-vis de cellules bactériennes.

5. Procédé selon la revendication 4, dans lequel les ligands non spécifiques comprennent des lectines, des immunoglobulines de mammifères, des peptides antimicrobiens, des polymères à base de carbohydrate(s) ou des constructions macromoléculaires biomimétiques.

6. Procédé selon l'une quelconque des revendications précédentes, dans lequel la procédure de lecture électrochimique comprend un procédé ampérométrique, l'électrode de détection étant utilisée pour mesurer un courant, en association avec une électrode de référence et/ou une contre-électrode.

7. Procédé selon la revendication 6, dans lequel la procédure de lecture électrochimique comprend une voltamétrie en mode stationnaire, une voltamétrie à balayage linéaire ou une voltamétrie à impulsion(s) différentielle(s).

8. Procédé selon la revendication 6 ou 7, dans lequel l'électrode de détection est une électrode de détection prise parmi : une électrode en graphite ou en carbone imprimée par sérigraphie, une électrode à nanotubes en carbone imprimée par jet d'encre, une microélectrode en carbone, en or ou en platine, par exemple réalisée par photolithographie.

9. Procédé selon l'une quelconque des revendications précédentes, dans lequel l'échantillon est un échantillon pris parmi : du sang, du sérum, du plasma, de l'urine, une substance issue de l'expectoration, de la salive ou un fluide interstitiel ; selon une forme pure, une forme diluée ou n'importe quelle autre forme liquide traitée.

10. Procédé selon l'une quelconque des revendications 1 à 8, dans lequel l'échantillon est sélectionné parmi une culture de bactéries préparée en laboratoire, un échantillon prélevé dans l'environnement, un échantillon prélevé dans l'industrie, un échantillon de produit alimentaire ou un échantillon de boisson.

11. Procédé selon l'une quelconque des revendications précédentes, dans lequel le marqueur redox métabolique est un substrat soluble à l'eau pour une enzyme mitochondriale ou cytoplasmique qui est produite par des bactéries viables.

12. Procédé selon la revendication 11, dans lequel le marqueur redox métabolique est un marqueur redox métabolique pris parmi : un métallocène/alliage organométallique oxydé soluble à l'eau tel que le ferrocène-méthanol, un complexe organométallique tel que la tris-bipyridine d'osmium, un colorant redox tel qu'un sel de tétrazolium, de la résazurine, du bleu de méthylène ou du formazane.

13. Procédé selon l'une quelconque des revendications précédentes, dans lequel un ou plusieurs antibiotique(s) est/sont introduit(s) à l'intérieur de l'échantillon, lequel contient des bactéries, et dans lequel la modification de l'état d'oxydation du marqueur redox métabolique indique si les bactéries sont résistantes ou sensibles aux un ou plusieurs antibiotiques.

14. Procédé selon la revendication 13, dans lequel les un ou plusieurs antibiotiques sont sous forme solide, sous forme liquide ou sont solubilisés et introduits sur l'échantillon avant l'enrichissement/la séparation sur la base de l'affinité.

15. Procédé selon la revendication 14, dans lequel les un ou plusieurs antibiotiques sont appliqués sur du papier, sur de la nitrocellulose, sur un hydrogel ou sur un polymère.
